# EUROPÄISCHE PATENTSCHRIFT

(11) **EP 0 553 890 B1**
(45) Veröffentlichungstag und Bekanntmachung des Hinweises auf die Patenterteilung: **09.10.1996**
(21) Anmeldenummer: 93104118.0
(22) Anmeldetag: 01.02.1990
(51) Int. Cl.: C07C 65/24, A61K 31/195, A61K 31/19, C07C 217/48, C07C 217/58, C07C 217/76, C07C 229/36, C07C 229/60, C07C 233/25, C07C 237/30, C07C 317/22, C07C 323/16

(54) **Diphenylheteroalkylderivate, ihre Herstellung und daraus hergestellte Arzneimittel und Kosmetika**
Diphenyl heteroalkyl derivatives, their preparation, as well as medicinal and cosmetic preparations prepared therefrom
Dérivés diphényl hétéroalkyles, leur préparation ainsi que médicaments et cosmétiques préparés à partir de ces composés

(30) Priorität: 10.02.1989 DE 3903989
(43) Veröffentlichungstag der Anmeldung: 04.08.1993
(62) Teilanmeldung aus: 90101944.8
(73) Patentinhaber: BASF Aktiengesellschaft, 67063 Ludwigshafen (DE)
(72) Erfinder: Janssen, Bernd, Dr., W-6700 Ludwigshafen (DE); Wuest, Hans-Heiner, Dr., W-6915 Dossenheim (DE)

(56) Entgegenhaltungen:
- EP-A- 0 170 105
- EP-A- 0 276 065
- DE-A- 3 524 199
- FR-A- 2 413 382
- FR-A- 2 629 817
- CHEMICAL AND PHARMACEUTICAL BULLETIN, Bd. 34, Nr. 5, Mai 1986, Tokyo, JP, Seiten 2275 - 2278, H. KAGECHIKA et al: "Differentiation inducers of human promyelocytic leukemia cells HL-60. Phenylcarbamoylbenzoic acids and polyene amides"
- JOURNAL OF MEDICINAL CHEMISTRY, Bd. 31, Nr. 11, November 1988, Washington, US, Seiten 2182 - 2192, H. KAGECHIKA et al: "Retinobenzoic acids. 1. Structure-activity relationships of aromatic amides with retinoidal activity"

## Beschreibung

Es ist bekannt, daß Stilbenderivate [vgl. DE-OS 2 854 354, DE-OS 3 534 564 und EP 212-137 (US 4 588 750)], bei welchen die Polyenstruktur von Substanzen des Vitamin-A-Typs in aromatischen Ringen fixiert vorleigt, pharmakologische Wirkungen bei der topischen und systemischen Therapie von Neoplasien, Akne, Psoriasis und anderen dermatologischen Affektionen aufweisen. Die Wirkung dieser Verbindungen befriedigt jedoch nicht immer [vgl. G.L. Peck in: The Retinoids, Vol. II, 391-409, Ed.: M.B. Sporn et al., Academic Press N.Y. (1984), oder R. Marks et al., Med. J. Austraiia 146, 374-377 (1987), oder C.E. Orfanos et al., Drugs 34, 459-503 (1987)].

In der FR-A-2 413 382 werden als Arzneimittel zur Behandlung von Arteriosklerose Ether der allgemeinen Formel 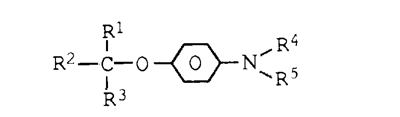 in der R¹ Wasserstoff, R² Phenyl oder Benzyl and R³, R⁴ und R⁵ Wasserstoff bedeuten können, beschrieben.

Aus der EP-A 0 276 065 sind anti-inflammatorisch wirkende Verbindungen der allgemeinen Formel 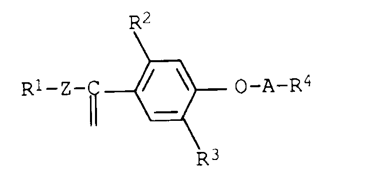 bekannt, wobei unter anderem R¹ Wasserstoff, Z Alkylen-, A C₁-C₁₀-Alkylen- und R⁴ einen gegebenenfalls mono- oder disubstituierten Phenylrest bedeuten kann.

Weiterhin ist bekannt (J. Med. Chem., 31, 11, S. 2182-92, 1988), daß Retinoide aus der Gruppe der Terephthalsäuremonoanilide und der(Arylcarboxamido)benzoesäuren mit retinoidaler Aktivität potentiell für die klinische Anwendung auf dem Gebiet der Dermatologie und der Onkologie geeignet sind.

Der Erfindung lag daher die Aufgabe zugrunde, Verbindungen mit bessarem Wirkspektrum zu entwickeln.

Überraschend wurde nun gefunden, daß Diphenylheteroalkylderivate der Formel I 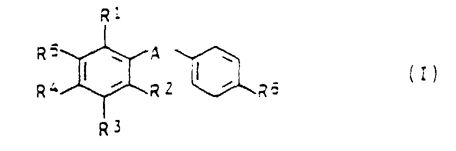 in der
- A: eine -E-CH₂- oder 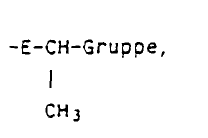 wobei E mit dem linken oder dem rechten Phenylkern verknüpft sein kann und für ein Sauerstoffatom, eine -S(O)ₙ- oaer -NR⁷-Gruppe steht (mit n in der Bedeutung von 0, 1 oder 2),
- R¹, R² und R³: unabhängig voneinander ein Wasserstoff- oder Halogenatom, eine C₁-C₆-Alkylgruppe oder eine OR⁷-Gruppe, einer der drei Reste auch eine Nitrogruppe,
- R⁴ und R⁵: unabhängig voneinander ein Wasserstoffatom, eine OR⁷-Gruppe oder eine C₁₋₆-Alkylgruppe,
- R⁶: ein Wasserstoffatom, eine Methyl-, eine Nitro- oder Nitrilgruppe, den Tetrazolylrest oder die Reste -CH₂OR⁷, -OR⁸, -NR⁹R¹⁰, -CH₂NR⁹R¹⁰, -CH(OR¹¹)_{2,} -SR¹⁰, -S(O)ₙR¹² (n = 1,2), -PO(OR¹³)₂, -NR¹³OR¹⁹, -SO₃H oder -C(O)R¹⁴ bedeuten, worin
- R⁷: ein Wasserstoffatom, eine C₁₋₆-Alkyl- oder C₁₋₆-Alkanoylgruppe,
- R⁸: ein Wasserstoffatom, eine C₁₋₆-Alkyl-, eine C₁₋₆-Alkanoyl- oder gegebenenfalls substituierte Benzoylgruppe oder einen -CH₂-C(O)R¹⁵-Rest (mit R¹⁵ in der Bedeutung von Wasserstoff, einer C₁₋₆-Alkyl-, einer C₁₋₆-Alkanoyl- oder einer Hydroxy-Gruppe oder des Restes -NR¹⁶R¹⁷),
- R⁹ und R¹⁰: unabhängig voneinander ein Wasserstoffatom, eine C₁₋₄-Alkyl-, C₁₋₄-Alkanoyl- oder eine gegebenenfalls durch Hydroxy oder C₁₋₄-Alkoxy substituierte Benzyl- oder Benzoylgruppe,
- R¹¹: eine C₁₋₆-Alkoxygruppe, wobei beide Reste R¹¹ unter Einschluß der CH-Gruppe gegebenenfalls ein cyclisches Acetal bilden,
- R¹²: eine C₁₋₆-Alkylgruppe,
- R¹³ und R¹⁹: unabhängig voneinander ein Wasserstoffatom oder eine C₁₋₃-Alkylgruppe;
- R¹⁴: ein Wasserstoff- oder Halogenatom, eine Hydroxy-, eine C₁₋₆-Alkyl-, eine C₁₋₆-Alkoxy- oder gegebenenfalls durch Hydroxy oder C₁₋₄-Alkoxy substituierte Phenoxy- oder Benzyloxygruppe oder den Rest -NR¹⁶R¹⁷ mit R¹⁶ und R¹⁷ unabhängig voneinander in der Bedeutung Wasserstoffatom, C₁₋₄-Alkylgruppe oder gegebenenfalls durch Hydroxy oder C₁₋₄-Alkoxy substituierte Benzylgruppe darstellen,
mit dem Proviso, daß R⁶ ≠ H, CO₂H, CO₂Alkyl, Alkyl, CN, Tetrazol, wenn R³ und R⁵ = tert.-Butyl und X mit dem linken Phenylkern verknüpft ist, und daß R⁶ ≠ CO₂H, wenn R¹ bis R³ und R⁵ = H und X mit dem rechten Phenylkern verknüpft ist, und daß R¹ bis R⁵ nicht alle gleichzeitig Wasserstoff darstellen.

sowie gegebenenfalls deren physiologisch verträglichen Salze ein besseres Wirkspektrum besitzen.

Von den Verbindungen der Formel I sind diejenigen bevorzugt, in denen A die oben angegebene Bedeutung hat und X für eine Ether-, Thioether- oder NH-Brücke steht.

Sind R¹, R² und/oder R³ Halogenatome, so sind Fluor und Chlor bevorzugt.

Stellen R⁴ und/oder R⁵ C₁₋₆-Alkylgruppen dar, so sind verzweigte gegenüber linearen Alkylresten bevorzugt.

Bevorzugt sind ferner Verbindungen der Formel I, in denen R⁶ für einen der Reste -CH₂OR⁷, -OR⁸, -SR¹⁰, -S(O)₂R¹², SO₃H, -PO(OR¹³)₂ oder -C(O)R¹⁴ steht; darunter werden solche Verbindungen besonders bevorzugt, in denen R⁷ Wasserstoff, R⁸ Wasserstoff, eine Acetylgruppe oder den Benzoylrest, der vorzugsweise mit Amino-, Acetamino-, Dimethylamino-, Hydroxy-, Methoxy- oder Methylgruppen oder Halogenatomen, insbesondere Fluor oder Chlor, substituiert ist, bzw. R¹⁰ Wasserstoff, eine Acetylgruppe oder den Benzoylrest, der vorzugsweise mit Amino-, Acetamino-, Dimethylamino-, Methoxy- oder Methylgruppen oder Halogenatomen, insbesondere Fluor oder Chlor, substituiert ist, R¹² eine Methyl- oder Ethylgruppe, R¹³ und R¹⁹ Wasserstoff oder eine Methylgruppe, R¹⁴ Wasserstoff, eine Hydroxy-, Methyl-, Methoxy-, Ethoxy- oder Phenoxygruppe, die gegebenenfalls durch Amino-, Acetamino-, Dimethylamino-, Hydroxy- oder Methoxygruppen substituiert ist, oder den Rest -NR¹⁶R¹⁷ mit R¹⁶ und/oder R¹⁷ vorzugsweise in der Bedeutung von Wasserstoff, der Methyl-oder Benzylgruppe, die gegebenenfalls durch Acetoxy-, Hydroxy- oder Methoxygruppen substituiert ist, oder mit R¹⁶ in der Bedeutung von Wasserstoff und R¹⁷ in der Bedeutung einer OH-Gruppe, bedeutet.

Die neuen Verbindungen der Formel I enthalten teilweise chirale Zentren und fallen im allgemeinen als Diastereomerengemische, bzw. Racemate an. Die so erhaltenen Diasteromeren lassen sich beispielsweise durch Löslichkeitsunterschiede oder durch Säulenchromatographie trennen und in reiner Form isolieren. Aus den Enantiomerenpaaren kann man nach bekannten Methoden einheitliche Enantiomere erhalten. Sowohl diese als auch ihre Gemische (Racemate) werden von der vorliegenden Erfindung umfaßt. Als therapeutische oder kosmetische Mittel kann man sowohl die einheitlichen Diastereomeren bzw. Enantiomeren wie auch deren Gemische verwenden.

Einige der erfindungsgemäßen Verbindungen weisen ein acides Wasserstoffatom auf und können daher mit Basen in üblicher Weise in ein physiologisch verträgliches, gut wasserlösliches Salz überführt werden. Geeignete Salze sind beispielsweise Ammonium- und Alkalimetallsalze, insbesondere des Natriums, Kaliums und Lithiums, oder Erdalkalimetallsalze, insbesondere des Calciums oder Magnesiums, sowie Salze mit geeigneten organischen Basen, wie mit niederen Alkylaminen, z. B. Methylamin, Ethylamin oder Cyclohexylamin, oder mit substituierten niederen Alkylaminen, insbesondere hydroxysubstituierten Alkylaminen, wie Diethanolamin, Triethanolamin oder Tris-(hydroxymethyl)-aminomethan sowie mit Piperidin oder Morpholin.

Gegebenenfalls werden die erhaltenen erfindungsgemäßen Amine der Formel (I) nach bekannter Verfahrensweise in das Säureadditionssalz einer physiologisch verträglichen Säure übergeführt. Als übliche physiologisch verträgliche organische oder anorganische Säure kommen beispielsweise in Betracht: Salzsäure, Bromwasserstoffsäure, Phosphorsäure oder Schwefelsäure und als organische Säuren beispielsweise Maleinsäure, Fumarsäure, Milchsäure, Weinsäure, Äpfelsäure, Zitronensäure, Salicylsäure, Adipinsäure oder Benzoesäure. Weitere können aus "Fortschritte der Arnzeimittelforschung" Band 10, Seiten 224-225, Birkhäuser Verlag, Basel und Stuttgart, 1966, entnommen werden.

Gegenstand der vorliegenden Erfindung ist weiter ein Verfahren zur Herstellung der oben angegebenen Verbindungen der Formel I, indem man
a)
   - falls A eine -O-CH₂ oder -O-CH(CH₃)-Gruppe bedeutet - Phenole der Formel II 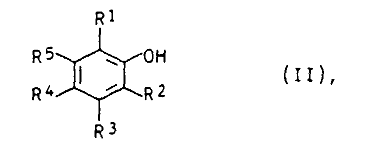 in der R¹-R⁵ die oben angegebenen Bedeutungen haben, mit Benzylderivaten der Formel III 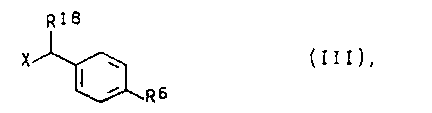 in der R⁶ die oben angegebenen Bedeutungen haben kann, R¹⁸ Wasserstoff oder Methyl und X eine nucleofuge Abgangsgruppe darstellt, umsetzt, oder
b)
   - falls A eine -CH₂-O- oder -CH(CH₃)-O-Gruppe bedeutet - Phenole der Formel IV 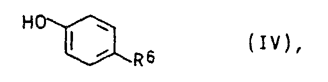 in der R⁶ die oben angegebene Bedeutung hat, mit Benzylderivaten der Formel V 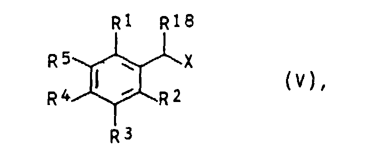 in der R¹-R⁵, R¹⁸ und X die oben angegebenen Bedeutungen haben, umsetzt, oder
c)
   - falls A eine -S(O)ₙ-CH₂- oder S(O)ₙ-CH(CH₃)-Gruppe bedeutet - Thiophenole der Formel VI 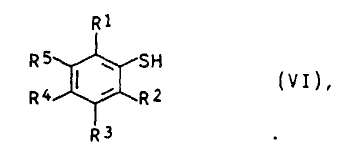 in der R¹-R⁵ die oben angegebenen Bedeutungen haben, mit Benzylderivaten der Formel III zu Thioethern (n=0) umsetzt und diese gegebenenfalls zu den korrespondierenden Sulfoxiden (n=1) oder Sulfonen (n=2) oxidiert, oder
d)
   - falls A eine -CH₂-S(O)ₙ- oder -CH(CH₃)-S(O)ₙ-Gruppe bedeutet - Thiophenole der Formel VII 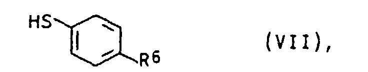 in der R⁶ die oben angegebene Bedeutung hat, mit Benzylderivaten der Formel V zu Thioethern (n=0) umsetzt und diese gegebenenfalls in die korrespondierenden Sulfoxide (n=1) oder Sulfone (n=2) überführt,
e)
   - falls A eine -NR⁷CH₂- oder -NR⁷CH(CH₃)-Gruppe bedeutet - entweder
      1. Aniline der Formel VIII 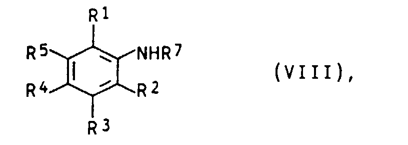 in der R¹-R⁵ und R⁷ die oben angegebenen Bedeutungen haben, mit Benzylderivaten der Formel III wie unter a) angegeben umsetzt, oder
      2. Aniline der Formel VIII mit Carbonylverbindungen der Formel IX 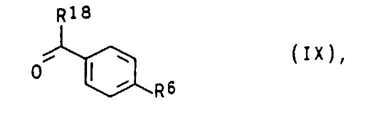 in der R⁶ und R¹⁸ die oben angegebenen Bedeutungen haben, in Gegenwart eines Reduktionsmittels umsetzt;
f)
   - falls A eine -CH₂NR⁷- oder -CH(CH₃)NR⁷-Gruppe bedeutet - entweder

   1. Aniline der Formel X 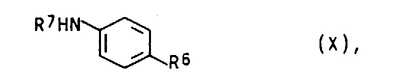 in der R⁶ und R⁷ die oben angegebenen Bedeutungen haben, mit Benzylderivaten der Formel V wie unter b) angegeben umsetzt, oder
   2. Aniline der Formel X mit Carbonylverbindungen der Formel XI 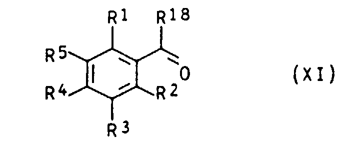 mit R¹-R⁵ und R¹⁸ in der oben angegebenen Bedeutung in Gegenwart eines Reduktionsmittels umsetzt.

Die Alkylierungsreaktionen a)-d) sowie el) und fl) erfolgen in an sich bekannter Weise, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- oder Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Als nucleofuge Abgangsgruppen kommen vorzugsweise in Betracht: Brom, Chlor, die Methylsulfonyloxy-, Trifluormethylsulfonyloxy- und die Toluolsulfonyloxygruppe.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid; Alkalicarbonate wie Natrium- oder Kaliumcarbonat oder Natrium- und Kaliumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyl-tri-ethylammoniumchlorid oder -bromid oder Kronenether wie 12-Krone-4, 15-Krone-5, 18-Krone-6, Di-benzo-18-Krone-6 oder Dicyclohexano-18-Krone-6 in Frage.

Die Umsetzung wird im allgemeinen bei Temperaturen zwischen 20 und 150°C, drucklos oder unter Druck, kontinuierlich oder diskontinuierlich durchgeführt.

Die unter c) und d) angeführten Oxidationsreaktionen der Thioether erfolgen gegebenenfalls in Gegenwart eines Lösungs- oder Verdünnungsmittels und gegebenenfalls unter Zusatz eines Katalysators, indem man 1.0 Äquivalent des Thioethers mit 1.0 bis 1.1 Äquivalenten des Oxidationsmittels bei Temperaturen von -30 bis 120°C zu dem entsprechenden Sulfoxid umsetzt.

Die Darstellung der entsprechenden Sulfone erfolgt durch Verwendung von 2.0 bis 3.0 Äquivalenten an Oxidationsmittel. Zu den bevorzugten Lösungs- oder Verdünnungsmitteln gehören niedere Alkylcarbonsäuren wie Ameisensäure, Essigsäure und Propionsäure, Alkohole wie Methanol, Ethanol oder Isopropanol, Kohlenwasserstoffe wie Hexan, Cyclohexan oder Heptan, Aromaten wie Benzol, Toluol oder Xylol, Ether wie Methyl-tert.-butylether, Diisopropylether oder Diphenylether, Ketone wie Aceton oder Methylethylketon halogenierte Kohlenwasserstoffe wie Dichlormethan, Chloroform, Tetrachlorkohlenstoff, Dichlorethan oder Chlorbenzol, aber auch Nitrile wie Acetonitril und Propionitril oder Amide wie Dimethylformamid, Dimethylacetamid oder Pyrrolidon sowie Wasser; es sind aber auch Gemische derselben geeignet.

Als Oxidationsmittel kommen in Frage:
Peroxiverbindungen wie Wasserstoffperoxid, tert.Butylhydroperoxid, Peressigsäure, Perbenzoesäure, mono-Perphthalsäure oder halogenierte Perbenzoesäuren wie m-Chlorperbenzoesäure. Es sind aber auch andere Oxidationsmittel wie Kaliumpermanganat, Kaliumdichromat, Natriumperjodat oder Perjodsäure sowie Salpetersäure oder Nitrosegase wie Stickstoffdioxid verwendbar (vgl. beispielsweise "Methoden der Organischen Chemie" Hrsg. Eugen Müller, Bd. IX, S. 207 ff und S. 223 ff, Thieme Verlag, Stuttgart 1955, sowie Reid, "Organic Compounds of Bivalent Sulfur", Bd. 2, S. 64 ff, Chem. Publ. New York, 1960).

Als Katalysatoren kommen gegebenenfalls in Betracht Mineralsäuren wie Chlorwasserstoffsäure oder Schwefelsäure, aber auch Alkalihydroxide wie Natrium- oder Kaliumhydroxid sowie Alkalicarbonate wie Natrium- oder Kaliumcarbonat.

Im Falle, daß die Reaktion in einem zweiphasigen System abläuft, können Phasentransferkatalysatoren, z. B. quarternäre Ammoniumverbindungen wie Tetrabutylammoniumsalze, zur Reaktionsbeschleunigung verwendet werden.

Die Alkylierungsreaktionen e2) und f2) erfolgen in an sich bekannter Weise im Sinne einer reduktiven Alkylierung, gegebenenfalls in Gegenwart eines Lösungs- oder Verdünnungsmittels, gegebenenfalls unter Zusatz eines Reaktionsbeschleunigers. Die Umsetzung wird im allgemeinen bei Temperaturen zwischen 20 und 150 ^{o}C, drucklos oder unter Druck, kontinuierlich oder diskontinuierlich durchgeführt.

Die Anilin- und die Carbonylkomponente werden im Verhältnis 0.5:1 bis 2:1, vorzugsweise in äquimolarem Verhältnis eingesetzt.

Zu den bevorzugten Lösungsmitteln gehören Kohlenwasserstoffe wie Heptan, Cyclohexan, Toluol oder Xylol, ferner Ether wie Diethylether, Tetrahydrofuran oder Dioxan, Halogenkohlenwasserstoffe wie Dichlormethan, Trichlormethan oder Chlorbenzol, Alkohole wie Methanol, Ethanol, Isopropanol oder Cyclohexanol, Alkyl-Carbonsäuren wie Ameisensäure, Essigsäure oder Propionsäure, ferner Acetonitril, Wasser oder entsprechende Gemische.

Als Reduktionsmittel kommen in Frage, Ameisensäure, Wasserstoff oder Metallhydride wie Lithiumaluminiumhydrid, Natriumborhydrid oder Natriumcyanoborhydrid.

Es sind aber auch andere Reduktionsmittel einsetzbar [vgl. "Methoden der Organischen Chemie", Hrsg. Eugen Müller, Bd. XI/1, S. 618 ff, S. 648 ff, S. 669 ff, Thieme Verlag, Stuttgart 1957, oder Watanabe et al. Tetrahedron Lett. 1879 (1974)].

Als Hydrierkatalysatoren in homogener oder heterogener Phase kommen bevorzugt in Betracht Nickel, Cobalt oder Platinkatalysatoren (vgl. Markò et al., J. Organomet. Chem. 81, 411 (1974), bzw. Rylander, "Catalytic Hydrogenation over Platinum Metals", S. 291-303, Academic Press, N.Y., 1967).

Die Ausgangsverbindungen der Formel II sind bekannt oder können nach den allgemein üblichen Methoden zur Herstellung substituierter Phenolderivate erhalten werden ("Methoden der Organischen Chemie", Hrsg. Eugen Müller, Bd. VI/1c, S. 4 ff, S. 313 ff, S. 925 ff, Thieme Verlag, Stuttgart, 1976).

Die Benzylderivate der Formel III, worin X für ein Brom- oder Chloratom steht, sind bekannt oder lassen sich darstellen aus den entsprechenden Alkylbenzolderivaten (X=Wasserstoff) durch Halogenierung in an sich bekannter Weise ("Methoden der Organischen Chemie", Hrsg. Eugen Müller, Bd. V/3, S. 735 ff, S. 809, Thieme verlag, Stuttgart, 1962 und Bd. V/4, S. 219 ff, Thieme Verlag, Stuttgart, 1960; W. Foerst: "Neuere Methoden der präparat. org. Chemie", Bd. III, S. 134, Verlag Chemie, Weinheim, 1961).

Benzylderivate der Formel III, worin X für eine OH-Gruppe steht, sind teilweise bekannt oder werden hergestellt durch Reduktion der korrespondierenden Carbonylderivate der Formel IX. Die Darstellung der reaktiven Ester der Formel III, worin X eine nucleofuge Abgangsgruppe bedeutet, erfolgt hieraus nach allgemein bekannten Methoden (Houben-Weyl-Muller, Methoden der organischen Chemie, Georg Thieme Verlag, Stuttgart, 1955, Band IX, Seiten 388, 663, 671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Ausgangsverbindungen der Formel IV sind bekannt oder werden nach allgemein bekannten Verfahren zur Herstellung substituierter Phenole erhalten, entsprechend den zur Herstellung von Verbindungen des Typs II oben angegebenen Methoden.

Die Benzylderivate der Formel V, worin X für ein Brom- oder Chloratom steht, sind bekannt. Sie lassen sich herstellen aus den entsprechenden Alkylbenzolderivaten (X=Wasserstoff) durch Halogenierung in an sich bekannter Weise (s. Literaturhinweis bei Ausgangsstoffen der Formel III).

Verbindungen der Formel V, worin-X eine reaktive Estergruppe bedeutet, werden entsprechend dem für die Vorstufen der Formel III oben angegebenen Weg erhalten, indem man die korrespondierenden Carbonylverbindungen der Formel XI reduziert und anschließend in die reaktiven Ester überführt.

Die Ausgangsverbindungen der Formel VI sind bekannt oder lassen sich nach den für die Darstellung von Thiophenolen üblichen Verfahren erhalten (K.-D. Gundermann und K. Hümke in "Methoden der Organischen Chemie", Bd. Ell, S. 32 ff, Thieme Verlag, Stuttgart, 1985 und dort zitierte Literatur). Sie lassen sich bevorzugt herstellen durch Reduktion der entsprechenden Sulfonsäurederivate, zum Beispiel mit Metallhydriden, oder auch aus den entsprechenden Phenolen der Formel II, die zu Thiocarbaminsäureestern umgesetzt werden (Newman u. Karnes, J. Org. Chem. 31, 3980 (1966)).

Die Ausgangsverbindungen der Formel VII sind bekannt oder lassen sich nach allgemein üblichen Verfahren - wie für die Thiophenole der Formel VI oben beschrieben - herstellen.

Die Ausgangsverbindungen der Formel VIII sind bekannt oder werden in an sich bekannter Weise hergestellt, beispielsweise durch Reduktion der entsprechenden Nitroverbindungen ("Methoden der Organischen Chemie", Hrsg. E. Müller, Bd. XI/1, S. 394, Thieme-Verlag, Stuttgart, 1957).

Die Ausgangsverbindungen der Formel IX sind bekannt oder werden erhalten nach üblichen Methoden zur Darstellung von Acetophenonen, beispielsweise durch Friedel-Crafts Acylierung (H.O. House: "Modern Synthetic Reactions", 2nd Ed., W.A. Benjamin Inc. Menlo Park, CA, (1972), S. 797 ff und dort zitierte Literatur) oder durch Oxidation der entsprechenden Alkylbenzole (H.O. House. l.c., S. 288 f und dort angegebene Literatur) sowie zur Darstellung von Benzaldehyden, beispielsweise durch Aromatenformylierung nach Vilsmeier (vgl. De. Meheas, Bull. Soc. Chem. Fr. 1989-1999 (1962) und dort zitierte Literatur) oder durch Reduktion der entsprechenden Benzoylhalogenide (vgl. Fuson in: Patai, "The Chemistry of the Carbonyl Group", Vol. 1, S. 211- 232, Interscience Publ., N.Y. 1966 oder Wheeler in: Patai, "The Chemistry of Acyl Halides" S. 231-251, Interscience Publ. N.Y. 1972) oder Benzonitrile (vgl. J. March: "Advanced Organic Chemistry, 2nd Ed., McGraw-Hill Kogakusha LTD. Tokyo, 1977, S. 835-836 und dort zitierte Literatur).

Die Ausgangsverbindungen der Formel X sind bekannt oder werden hergestellt nach üblichen Methoden analog den oben bereits für die Darstellung von Anilinen des Typs VIII angegebenen.

Die Ausgangsverbindungen der Formel XI sind bekannt (z.B.: DE-OS 3 602 473, DE-OS 3 434 942, DE-OS 3 434 944) oder werden entsprechend den oben für die Herstellung von Carbonylverbindungen der Struktur IV angegebenen Verfahren erhalten.

Die erfindungsgemäßen Verbindungen der Formel I, in der A eine NR⁷-Gruppe, mit R⁷ in der Bedeutung von Wasserstoff, enthält, werden nach üblichen Methoden zur N-Alkylierung oder N-Acylierung in an sich bekannter Weise zu weiteren erfindungsgemäßen Verbindungen der Formel I umgesetzt.

Die nach den oben genannten Verfahren a-f hergestellten Substanzen lassen sich anschließend wie folgt weiter abwandeln:
Die Benzoesäureester der Formel I (R⁶=Carboalkoxy) werden, falls erwünscht, durch Esterverseifung in die freien Carbonsäuren übergeführt. Umgekehrt läßt sich natürlich die freie Säure in bekannter Weise verestern.

Zweckmäßigerweise wird die Verseifung/Veresterung in Gegenwart eines Verdünnungs- oder Lösungsmittels, beispeilsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon oder Methylisobutylketon, oder in einem niederen aliphatischen Alkohol, wie Methanol, Ethanol, Propanol oder Isopropanol, oder in Dimethylsulfoxid, gegebenenfalls in Gegenwart von Wasser bzw. in Mischungen der genannten Lösungsmittel mit Wasser durchgeführt.

Bevorzugte Lösungsmittel sind wäßrige Mischungen von Ethanol, Methanol und Dimethylsulfoxid, wobei die Umsetzung beim Siedepunkt des Reaktionsgemisches durchgeführt wird.

Die Verseifung geschieht bevorzugt in Gegenwart von Alkali, wie Alkalimetallhydroxide, Alkalimetallcarbonate und -hydrogencarbonate, insbesondere des Natriums und Kaliums, organischen tertiären Basen, wie Pyridin oder niedere Trialkylaminen, wie Trimethyl- oder Triethylamin, in Gemischen mit Wasser. Dabei wird die verwendete Base im Verhältnis zum Ester in stöchiometrischer Menge oder in geringem Überschuß eingesetzt. Vorzugsweise wird Natrium- oder Kaliumhydroxid verwendet.

Die Veresterung geschieht vorteilhaft, indem man die Carbonsäure zunächst in ihr Salz überführt und dieses mit einem entsprechenden Alkylhalogenid, vorzugsweise einem Alkylbromid oder -iodid, behandelt. Als Deprotonierungsmittel für die Herstellung der Salze in situ eignen sich insbesondere die Carbonate, Hydroxide und Hydride der Alkalimetalle. Zweckmäßigerweise verwendet man aprotische polare Lösungsmittel wie Aceton, Dimethylformamid, Dimethylsulfoxid und insbesondere Methylethylketon, wobei die Umsetzung beim Siedepunkt des Reaktionsgemisches durchgeführt wird.

Die erfindungsgemäßen Amide können in an sich bekannter Weise hergestellt werden, indem man die Benzoesäuren I (R⁶ = COOH) zunächst in carbonylaktivere Derivate überführt, z. B. in die Säurehalogenide, -azide, -imidazolide oder -anhydride, die O-Acyl-N,N'-dicyclohexylisoharnstoffe oder p-Nitrophenylester und diese mit Aminen HNR¹⁶R¹⁷ behandelt. In den Fällen besonders reaktiver Amine, vor allem Ammoniak, ist die direkte Amidolyse von Estern (mit dem Rest -C(O)R¹⁴ mit R¹⁴ in der Bedeutung von Alkoxy) bevorzugt.

Entsprechend wurden die erfindungsgemäßen Hydroxamsäure-Derivate der Formel I (R⁶ = CONR¹³OR¹⁹) aus den korrespondierenden Benzoesäuren I (R⁶ = CO₂H) hergestellt, indem man ihre carbonylaktivierten Derivate mit Hydroxylaminen des Typs NHR¹³OR¹⁹, vorzugsweise in einem polaren aprotischen Lösungsmittel wie Dimethylformamid, mit äquimolaren Mengen einer organischen oder anorganischen Base als Protonenfänger in einem zwischen Raumtemperatur und dem Siedepunkt des Reaktionsgemisches liegenden Temperaturbereich umsetzt. Die Reaktion wird drucklos oder unter Druck durchgeführt. Bevorzugt setzt man das Hydroxylamin in Form seines mineralsauren Salzes, insbesondere als Hydrochlorid ein, unter Verwendung eines weiteren Äquivalentes Base.

Eine Carbonsäure, ein Carbonsäureester oder ein Carbonsäureamid der Formel I (R⁶ = C(O)R¹⁴) kann in an sich bekannter Weise zu den entsprechenden Alkoholen bzw. Aminen reduziert werden. Vorteilhaft wird die Reduktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminiumhydrid oder Diisobutylaluminiumhydrid eingesetzt. Als Lösungsmittel werden beim Arbeiten mit Lithiumaluminiumhydrid Ether eingesetzt, wie Diethylether, Dioxan oder Tetrahydrofuran. Führt man die Reduktion mit Diisobutylaluminiumhydrid oder einem Alkoxynatriumaluminiumhydrid durch, so ist die Verwendung von Kohlenwasserstoffen wie Hexan oder Toluol bevorzugt.

So erhaltene Amine oder Alkohole können in an sich bekannter Weise mit einem Alkanoylhalogenid oder -anhydrid oder einem Aroylhalogenid oder -anhydrid zweckmäßigerweise in einem inerten Verdünnungs- oder Lösungsmittel, z. B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungs-oder Lösungsmittel in die erfindungsgemäßen Amide und Ester der Formel (I) überführt werden. Bevorzugt werden die Umsetzungen in Gegenwart einer Base als säurebindendem Mittel in einem zwischen -20^{o}C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Acylierungsmittel in katalytischer Menge oder in stöchiometrischer Menge bzw. in geringem Überschuß verwendet werden.

Ein unter die Formel I fallender Alkohol (R⁶ = CH₂OH) kann mit Alkylhalogeniden R⁷-J, R⁷-Br oder R⁷-Cl in Gegenwart von Alkalimetallhydriden, vorzugsweise Natriumhydrid, oder in Gegenwart von Alkyllithium-Verbindungen, vorzugsweise n-Butyllithium in einem organischen Lösungsmittel wie Tetrahydrofuran, Dioxan, 1,2-Dimethoxyethan, Methyl-tert.-butylether oder bei Verwendung von Natriumhydrid auch in Dimethylformamid in einem zwischen -10^{o}C und 40^{o}C liegenden Temperaturbereich zum entsprechenden Ether umgesetzt werden.

Ein unter die Formel I fallender Alkohol kann mit geeigneten Oxidationsmitteln, vorzugsweise Mangan(IV)-oxid, gegebenenfalls auf einem anorganischen Trägermaterial wie Silicagel oder Aluminiumoxid zum entsprechenden Aldehyd oxidiert werden. Vorteilhaft arbeitet man in einem inerten organischen Lösungsmittel, beispielsweise einem Kohlenwasserstoff wie Hexan oder in einem Ether wie Tetrahydrofuran oder in Mischungen der genannten Lösungs- und Verdünnungsmittel im Temperaturbereich zwischen -10°C und 30°C. Die benötigte Reaktionszeit ist im Wesentlichen von der Oxidationsaktivität des eingesetzten Mangan(IV)-oxids abhängig.

Einen Aldehyd I (R⁶ = -CHO) kann man auch durch Reduktion des entsprechenden Nitrils mit Diisobutylaluminiumhydrid in einem Lösungsmittel vorzugsweise in Toluol, Hexan, Tetrahydrofuran oder Mischungen dieser Lösungsmittel in einem Temperaturbereich zwischen -40^{o}C und Raumtemperatur erhalten.

Die unter die Formel I fallenden Aldehyde und Ketone werden auch dadurch erhalten, daß man ihre Ketale hydrolysiert, üblicherweise in Gegenwart einer Säure als Katalysator, vorzugsweise verdünnter Salz- oder Schwefelsäure, bei 20^{o}C oder bei bis zum Siedepunkt des Reaktionsgemisches erhöhter Temperatur. Zweckmäßigerweise arbeitet man in mit Wasser gemischten Lösungsmitteln wie Aceton, Dioxan, Tetrahydrofuran, vorzugsweise in kurzkettigen Alkoholen wie Methanol und Ethanol.

Ein Nitril der Formel I (R⁶ = -CN) kann in an sich bekannter Weise unter Säure- oder vorteilhafter Basenkatalyse zur entsprechenden Carbonsäure verseift werden. Als Basen bevorzugt sind Alkalimetallhydroxide, besonders Kaliumhydroxid, das im Überschuß eingesetzt wird. Als Lösungsmittel werden in der Regel mit Wasser mischbare Alkohole wie Methanol, Ethanol, Isopropanol oder n-Butanol eingesetzt. Die Umsetzung wird üblicherweise beim Siedepunkt des Reaktionsgemisches durchgeführt.

Aus den Nitrilen I (R⁶ = -CN) können durch Addition eines Azides, z. B. eines Alkalimetallazids, vorzugsweise Natriumazid, in Gegenwart von Aluminiumchlorid oder Ammoniumchlorid die entsprechenden Tetrazole erhalten werden. Als Lösungsmittel verwendet man bevorzugt cyclische Ether, wie Dioxan oder Tetrahydrofuran sowie insbesondere Dimethylformamid oder deren Mischungen, wobei die Umsetzung im allgemeinen bei einer Temperatur von 60-100^{o}C abläuft.

Die acylierten Phenole der allgemeinen Formel I (R⁶ = OCOCH₃) werden, falls dies erwünscht ist, in die freien Phenole und ihre physiologisch verträglichen Salze, durch Esterverseifung übergeführt. Zweckmäßigerweise erfolgt die Verseifung in Gegenwart eines Verdünnungsmittels, beispielsweise eines mit Wasser mischbaren Ethers, wie 1,2-Dimethoxyethan oder Tetrahydrofuran, oder eines niederen aliphatischen Alkohols, wie Methanol, Ethanol, Propanol, Isopropanol oder Butanol, gegebenenfalls in Gegenwart von Wasser bzw. in Mischungen der genannten Lösungsmittel. Bevorzugte Lösungsmittel sind wäßrige Mischungen von Ethanol oder Methanol, wobei die Umsetzung zwischen 20^{o}C und dem Siedepunkt des Reaktionsgemisches durchgeführt wird. Die Verseifung geschieht bevorzugt in Gegenwart von Hydroxiden oder Carbonaten der Alkali- und Erdalkalimetalle, insbesondere des Natriums und Kaliums.

Ein Phenol der Formel I kann in an sich bekannter Weise mit einem Alkanoylhalogenid oder -anhydrid, einem Aralkanoylhalogenid oder -anhydrid oder einem Aroylhalogenid oder -anhydrid zweckmäßigerweise in einem inerten Verdünnungs- oder Lösungsmittel, z.B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungsmittel oder Lösungsmittel in die erfindungsgemäßen Ester übergeführt werden. Bevorzugt werden die Umsetzungen in Gegenwart einer Base als säurebindendem Mittel in einem zwischen -20^{o}C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

Die Veretherung der Phenole der Formel I zu Arylethern der Formel I geschieht vorteilhaft, indem man das Phenol zunächst in sein Salz überführt und dieses mit einem entsprechenden Alkylhalogenid oder -sulfat, vorzugsweise einem Alkylchlorid, -bromid oder -jodid, behandelt. Als Deprotonierungsmittel für die Herstellung der Phenolate in situ eignen sich insbesondere die Carbonate, Hydroxide und Hydride der Alkalimetalle. Zweckmäßigerweise verwendet man aprotische polare Lösungsmittel wie Aceton, Dimethylformamid, Dimethylsulfoxid oder Methylethylketon, wobei die Umsetzung zwischen 20^{o}C und dem Siedepunkt des Reaktionsgemisches durchgeführt wird.

Die acylierten Thiophenole der allgemeinen Formel I (R⁶ = SCOCH₃) werden, falls dies erwünscht ist, in die freien Thiophenole und ihre physiologisch verträglichen Salze, durch Esterverseifung übergeführt. Zweckmäßigerweise erfolgt die Verseifung in Gegenwart eines Verdünnungsmittels, beispielsweise eines mit Wasser mischbaren Ethers, wie 1,2-Dimethoxyethan oder Tetrahydrofuran, oder eines niederen aliphatischen Alkohols, wie Methanol, Ethanol, Propanol, Isopropanol oder Butanol, gegebenenfalls in Gegenwart von Wasser bzw. in Mischungen der genannten Lösungsmittel. Bevorzugte Lösungsmittel sind wäßrige Mischungen von Ethanol oder Methanol, wobei die Umsetzung zwischen 20^{o}C und dem Siedepunkt des Reaktionsgemisches durchgeführt wird. Die Verseifung geschieht bevorzugt in Gegenwart von Hydroxiden oder Carbonaten der Alkali- und Erdalkalimetalle, insbesondere des Natriums und Kaliums.

Ein Thiophenol der Formel I kann in an sich bekannter Weise mit einem Alkanoylhalogenid oder -anhydrid, einem Aralkanoylhalogenid oder -anhydrid oder einem Aroylhalogenid oder -anhydrid zweckmäßigerweise in einem inerten Verdünnungs- oder Lösungsmittel, z.B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungsmittel oder Lösungsmittel in die erfindungsgemäßen Ester übergeführt werden. Bevorzugt werden die Umsetzungen in Gegenwart einer Base als säurebindendem Mittel in einem zwischen -20^{o}C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin oder niedere Trialkylamine, z.B. Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

Die Veretherung der Thiophenole der Formel I zu Arylthioethern der Formel I geschieht vorteilhaft, indem man das Thiophenol zunächst in sein Salz überführt und dieses mit einem entsprechenden Alkylhalogenid, oder -sulfat, vorzugsweise einem Alkylchlorid, -bromid oder -jodid, behandelt. Als Deprotonierungsmittel für die Herstellung der Thiophenolate in situ eignen sich insbesondere die Carbonate, Hydroxide und Hydride der Alkalimetalle. Zweckmäßigerweise verwendet man aprotische polare Lösungsmittel wie Aceton, Dimethylformamid, Dimethylsulfoxid oder Methylethylketon, wobei die Umsetzung zwischen 20^{o}C und dem Siedepunkt des Reaktionsgemisches durchgeführt wird.

Die erfindungsgemäßen Thioether der Formel I (R⁶ = SR¹²) werden, falls dies erwünscht ist, in die entsprechenden Sulfoxide (R⁶ = SOR¹²), bzw. Sulfone (R⁶ = SO₂R¹²) übergeführt. Die Oxidation zu Sulfoxiden erfolgt vorteilhaft, indem man den Thioether in alkoholischer Lösung mit äquimolaren Mengen oder einem bis zu 10-prozentigen Überschuß aus Perjodsäure oder einem Alkalisalz derselben, vorzugsweise dem Natriumsalz, bei Temperaturen von 0 bis 30^{o}C zur Reaktion bringt. Als Löslichkeitsvermittler sind beispielsweise Wasser, Dimethylsulfoxid oder Amide wie Dimethylformamid, aber auch Ketone wie Aceton geeignet. Die Oxidation zu Sulfonen erfolgt vorteilhaft, indem man 2.0 bis 3.0 Äquivalente des Oxidationsmittels bei Temperaturen von -30 bis 120°C, vorzugsweise bei -10 bis 60 °C auf den entsprechenden Thioether einwirken läßt. Als Oxidationsmittel eignen sich auch Wasserstoffperoxid und besonders Peroxicarbonsäuren, worunter m-Chlor-peroxibenzoesäure bevorzugt wird. Als Lösungsmittel werden bei der Verwendung von Wasserstoffperoxid vorzugsweise Essigsäure oder Acetonitril, bei der Verwendung von Peroxicarbonsäuren aprotische Lösungsmittel wie Methylenchlorid oder Toluol eingesetzt.

Die Thiophenole der Formel I (R⁶ = SH) können, falls dies erwünscht ist, in die korrespondierenden Sulfonsäuren übergeführt werden, indem man auf das jeweilige Thiophenol, vorteilhaft in Essigsäure bei Temperaturen von 10°C bis zum Siedepunkt der Reaktionslösung die 2- bis 5-fache molare Menge Wasserstoffperoxid einwirken läßt.

Aus den erfindungsgemäßen N-acylierten Aminen der Formel I (R⁶ = NHR¹⁰ mit R¹⁰ = C₁₋₄-Alkanoyl) erhält man durch saure oder alkalische Hydrolyse die korresponierenden Anilinderivate (R⁶ = NH₂). Zweckmäßigerweise wird die Hydrolyse in Gegenwart eines Lösungs- oder Verdünnungsmittels, beispielsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon, Methylisobutylketon, oder in einem niederen alipathischen Alkohol, wie Methanol, Ethanol, Propanol ocer Isopropanol, bzw. in Mischungen der genannten Lösungsmittel mit Wasser durchgeführt.

Bevorzugte Lösungsmittel sind wäßrige Mischungen von Ethanol und Methanol, wobei die Umsetzung beim Siedepunkt des Reaktionsgemisches durchgeführt wird.

Die alkalische Verseifung geschieht bevorzugt in Gegenwart von Alkali, wie Alkalimetallhydroxide, Alkalimetallcarbonate und -hydrogencarbonate, insbesondere des Natriums und Kaliums, organischen tertiären Basen, wie Pyridin oder niederen Trialkylaminen, wie Trimethyl- oder Triethylamin in Gemischen mit Wasser. Dabei wird die verwendete Base im Verhältnis zum Ester in stöchiometrischer Menge oder in geringem überschuß eingesetzt. Vorzugsweise wird Natrium-oder Kaliumhydroxid verwendet.

Die saure Verseifung erfolgt bevorzugt in Gegenwart von Mineralsäuren, wie Chlorwasserstoffsäure, Schwefelsäure oder Phosphorsäure, oder von organischen Säuren, wie Benzolsulfonsäure oder Toluolsulfonsäure. Dabei wird die verwendete Säure im Verhältnis zum Ester in stöchiometrischer Menge oder in geringem Überschuß eingesetzt. Vorzugsweise wird Chlorwasserstoffsäure verwendet.

So erhaltene Aniline (R⁶ = NH₂) können in an sich bekannter Weise mit einem Alkanoylhalogenid oder -anhydrid oder einem Aroylhalogenid oder -anhydrid oder einem Alkyl- oder Benzylhalogenid, zweckmäßigerweise in einem inerten Verdünnungs- oder Lösungsmittel, z.B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungs- oder Lösungsmittel in die erfindungsgemäßen Amine der Formel (I) (R⁶ = NR⁹R¹⁰) übergeführt werden. Bevorzugt werden die Umsetzungen in Gegenwart einer Base als säurebindendem Mittel in einem zwischen -20^{o}C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Acylierungs- oder Alkylierungsmittel in katalytischer Menge oder in stöchiometrischer Menge bzw. in geringem Überschuß verwendet werden.

Durch Hydrolyse der Phosphonsäurediester der Formel I (R⁶ = PO(OR¹³)₂) können in an sich bekannter Weise je nach Wahl der Hydrolysebedingungen die Phosphonsäuren oder Phosphonsäuremonoester hergestellt werden. Die Verseifung der Phosphonsäurediester mit wäßrigen Hydroxiden der Alkali-und Erdalkalimetalle - bevorzugt sind Natrium- und Kaliumhydroxid - führt allgemein zu den entsprechenden Phosphonsäuremonoestern. Die vollständige Hydrolyse gelingt durch Reaktion der Phosphonsäurediester mit Trialkylhalogensilanen, vorzugsweise Trimethylbrom- und Trimethyliodsilan, die vorteilhafterweise in situ aus Trimethylchlorsilan und einem Alkalimetallbromid bzw. -iodid hergestellt werden, und anschließender Behandlung mit Wasser oder verdünnten Mineralsäuren, z. B. Salzsäure oder Schwefelsäure.

Aus den so erhaltenen Säuren lassen sich weitere erfindungsgemäße Verbindungen nach bekannten Verfahrensweisen herstellen. So kann man eine Phosphonsäure der Formel I beispielsweise mit Phosphorpentachlorid in das Phosphonsäuredichlorid überführen, welches mit Alkoholen zu den entsprechenden Estern umgesetzt wird.

Aus Phosphonsäureestern oder Phosphonsäurechloriden können durch Umsetzung mit metallorganischen Reagentien, z. B. Grignardverbindungen, entsprechende Phosphanoxidderivate erhalten werden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und inneren Organe sowie bei der topischen und systemischen Therapie von Akne, Psoriasis und anderer mit pathologisch veränderter Verhornung einhergehenden dermatologischen Erkrankungen, insbesondere Ichthyosis, Dariersche Krankheit, Flechte, Leukoplakie, Ekzeme, aber auch gegen Vitiligo, Warzen, Lichtschäden (vorzeitige Alterung) der Haut, ferner gegen trockene Augen und andere Corneopathien sowie zur Behandlung von rheumatischen Erkrankungen, insbesondere solchen entzündlicher oder degenerativer Art, die Gelenke, Muskeln, Sehnen und andere Teile des Bewegungsapparates befallen, verwendet werden. Bevorzugte Indikationsgebiete sind: die Therapie von dermatologischen Erkrankungen; die Therapie von durch Sonnenlichteinwirkung bedingten Hautschädigungen; die Therapie von iatrogen, z.B. durch Corticosteroide induzierte Atrophie, bedingten Hautschädigungen und die prophylaktische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964-972 (1972) oder aus Nature 250, 64-66 (1974) und Nature 253, 47-50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Experimental Cell Research 117, 15-22 (1978) und Proc. Natl. Acad. Sci. USA 77, 2937-2940 (1980) entnommen werden.

Die antiarthritische Wirkung der erfindungsgemäßen Verbindungen kann in üblicher Weise im Tierexperiment im Adjuvans-Arthritis- oder Streptokokkenzellwandinduzierten-Arthritis-Modell bestimmt werden. Die dermatologische Aktivität, beispielsweise für die Behandlung von Akne, kann u.a. durch die komedolytische Aktivität und die Fähigkeit nachgewiesen werden, die Anzahl der Zysten im Modell der Rhino-Maus zu reduzieren.

Diese Methode ist von L.H. Kligman et al. in The Journal of Investigative Dermatology 73, 354-358 (1978) beschrieben.

Als weiteres Maß für die dermatologische Aktivität kann die Reduktion der Talgdrüsen und die damit einhergehende verminderte Talgproduktion am Seitenorgan des Hamsters dienen. Diese Methodik ist von E.C. Gomez in J.Am. Dermatol. 6, 746-750 (1982) beschrieben.

Ferner kann die durch die erfindungsgemäßen Verbindungen erzielbare Reversion von Hautschäden, welche durch UV-Licht ausgelöst wurden, in Tiermodellen bestimmt werden. Diese Methodik ist von L.H. Kligman et al. beschrieben in Connect. Tissue Res. 12, 139-150 (1984) und im Journal of the American Academy of Dermatology 15, 779-785 (1986).

Dementsprechend sind ein weiterer Gegenstand der Erfindung therapeutische Mittel zur topischen und systemischen Anwendung sowie kosmetische Mittel, die eine Verbindung der Formel (I) als Wirkstoff neben üblichen Trägerstoffen oder Verdünnungsmitteln enthalten.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen oder kosmetischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei systemischer Anwendung als therapeutische Mittel vorzugsweise in einer Einzeldosis von 0,1 bis 250 mg enthalten und täglich in einer oder mehreren Dosierungen je nach Art und Schwere der Erkrankungen verabreicht werden.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Zweckmäßige übliche Bestandteile von kosmetischen und pharmazeutischen Zubereitungen für die topische Anwendung sind beispielsweise:
anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere,
feste oder flüssige ölkomponenten bzw. Fettstoffe mineralischer, pflanzlicher oder tierischer Herkunft, synthetische Esteröle, wie Triglyceridester und Isopropylmyristat,
hydrophile Komponenten, wie Glycerin, Polyethylenglykol und Propylenglykol.

Weiterhin können als Inhaltsstoffe von Kosmetika genannt werden beispielsweise Lichtschutzmittel, Bräunungsmittel, Konservierungsmittel, Antioxidantien, Pigmente, Farbstoffe, etherische öle und Parfümöle, Vitamine, Pflanzenextrakte, Collagen usw. Diese Stoffe können beispielsweise dem CTFA, Cosmetic Ingredient Dictionary, 3. Auflage, Washington 1982, entnommen werden.

Die nachfolgenden Beispiele erläutern die Erfindung:

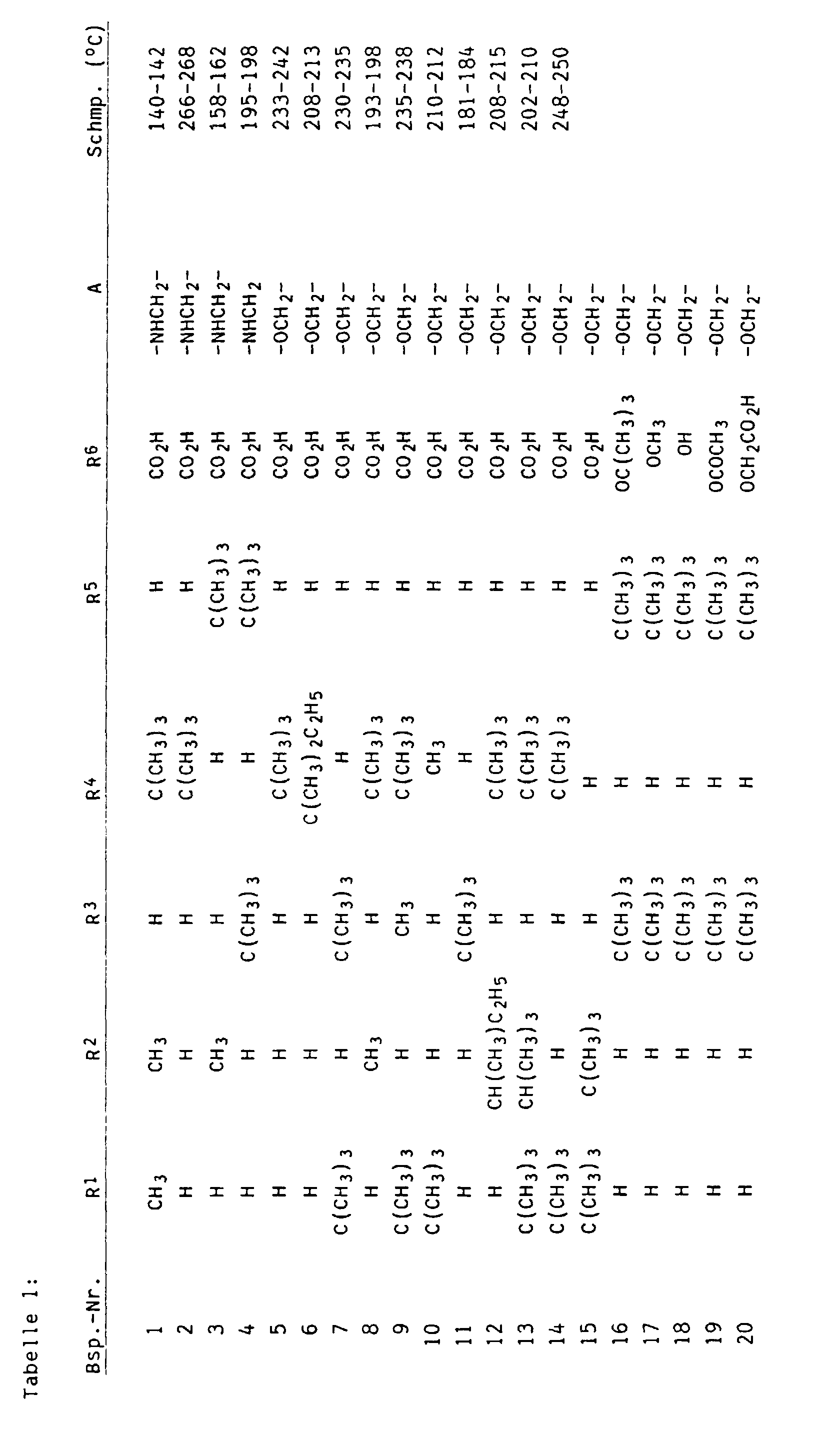

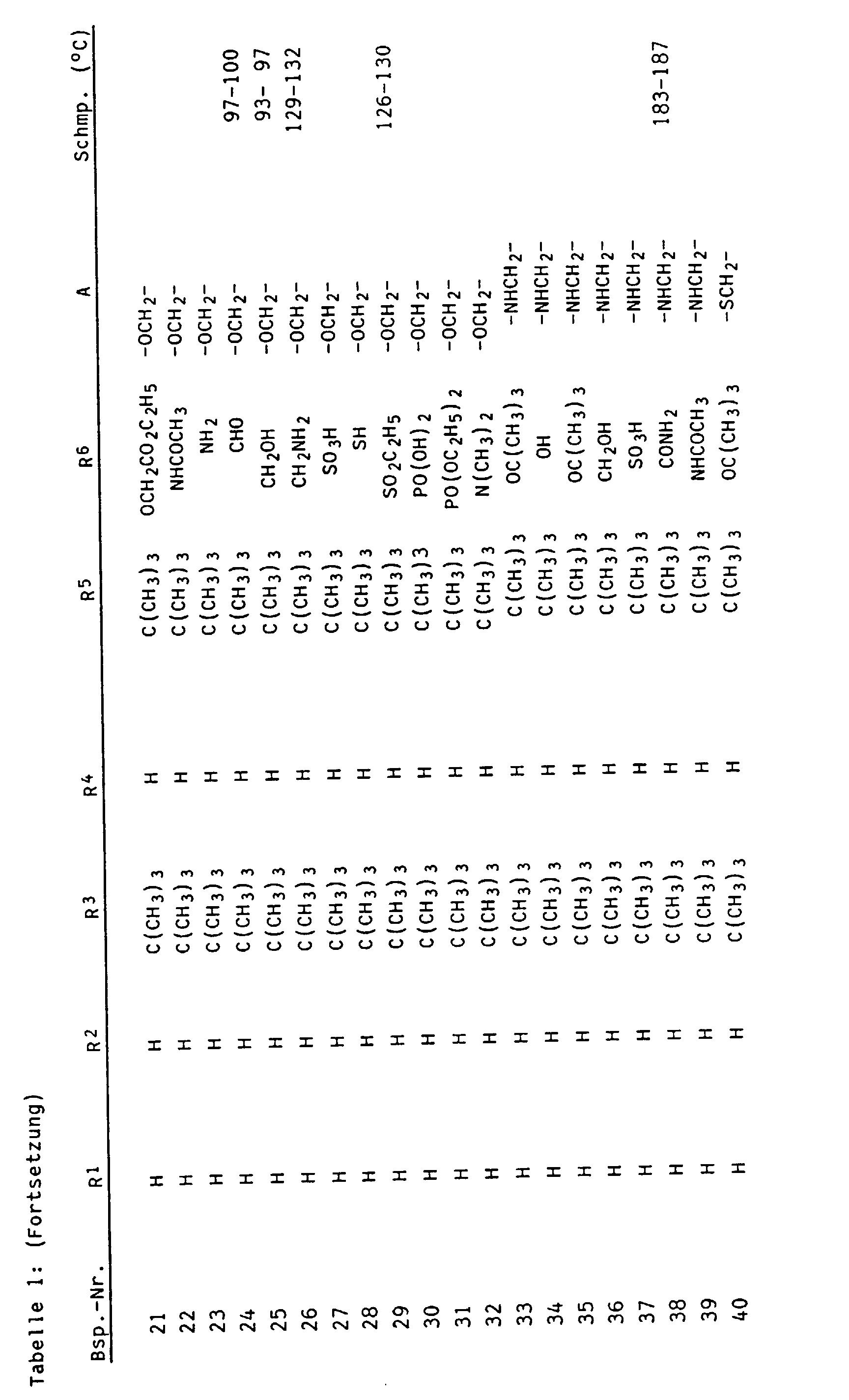

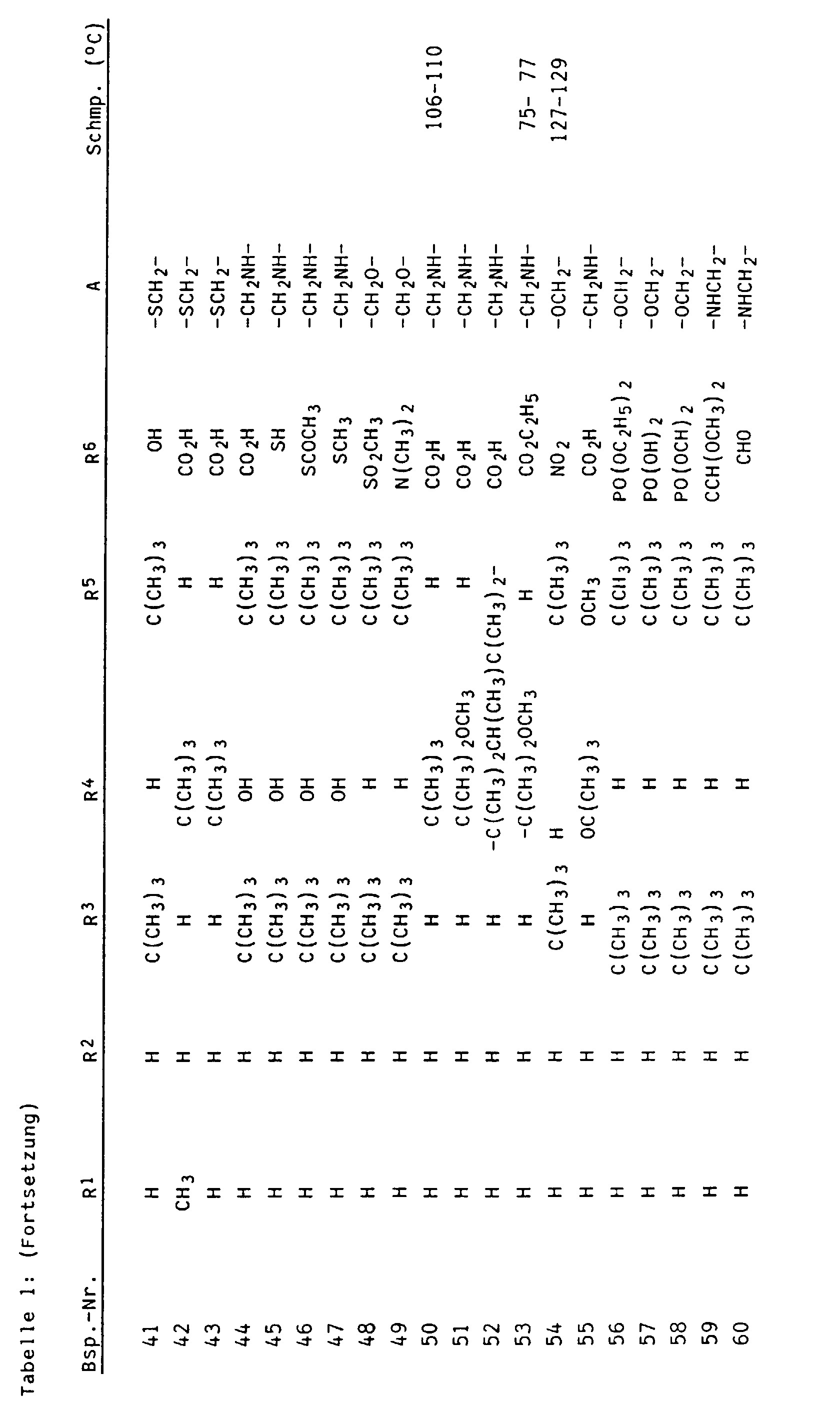

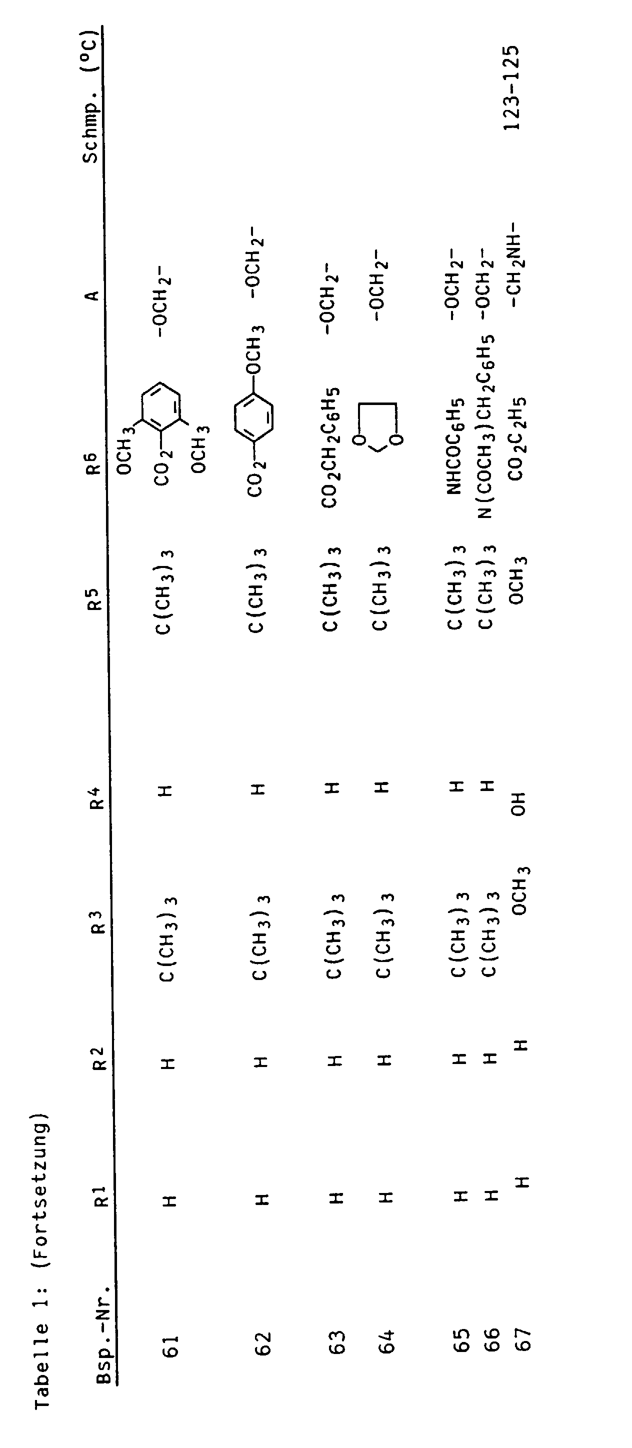

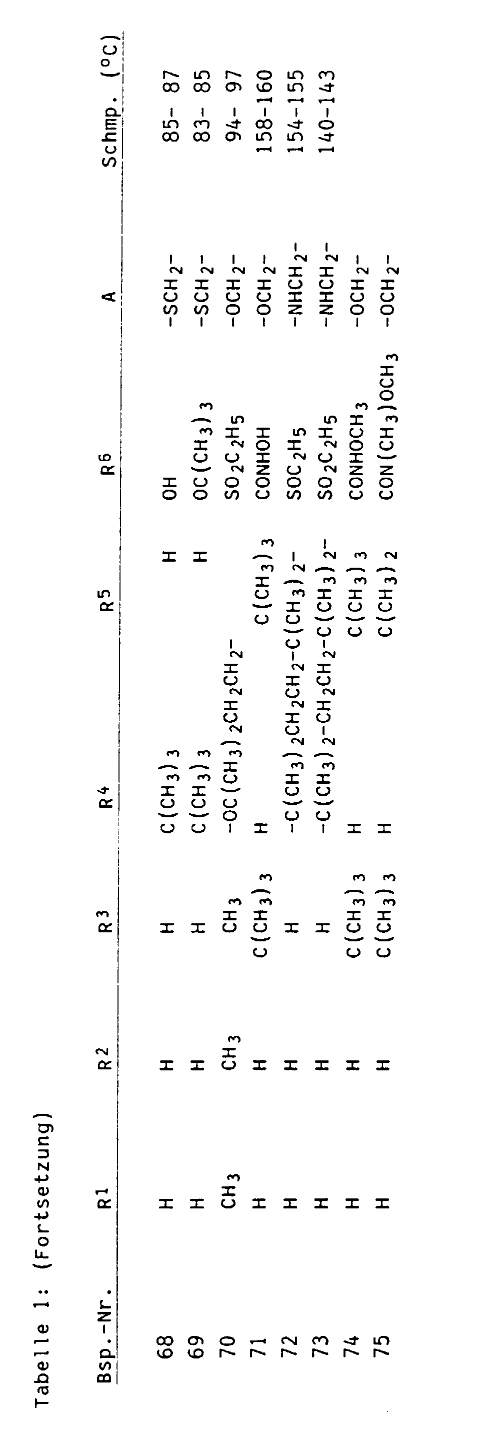

## Patentansprüche

1. Diphenylheteroalkylderivate der Formel I 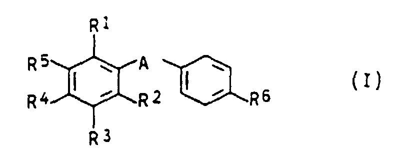 in der bedeuten:
A eine -E-CH₂- oder 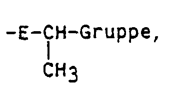 wobei E mit dem linken oder dem rechten Phenylkern verknüpft sein kann und für ein Sauerstoffatom, eine -S(O)ₙ- oder -NR⁷-Gruppe steht (mit n in der Bedeutung von 0, 1 oder 2),
R¹, R² und R³ unabhängig voneinander ein Wasserstoff- oder Halogenatom, eine C₁-C₆-Alkylgruppe oder eine OR⁷-Gruppe, einer der drei Reste auch eine Nitrogruppe,
R⁴ und R⁵ unabhängig voneinander ein Wasserstoffatom, eine OR⁷-Gruppe oder eine C₁₋₆-Alkylgruppe, wobei R⁴ eine verzweigte C₃₋₆-Alkyl- oder -Alkoxygruppe darstellt, wenn R¹ bis R³ Wasserstoff sind, und R⁴ (allein) oder R³ und R⁵ (beide) jeweils eine verzweigte C₃₋₆-Alkylgruppe darstellen, wenn R⁶ ein Wasserstoffatom, eine Methyl-, Nitro-, OH- oder SH-Gruppe ist,
R⁶ ein Wasserstoffatom, eine Methyl-, eine Nitro- oder Nitrilgruppe, den Tetrazolylrest oder die Reste -CH₂OR⁷, -OR⁸, -NR⁹R¹⁰, -CH₂NR⁹R¹⁰, -SR¹⁰, CH(R¹¹)₂, -S(O)ₙR¹² (mit n=1 oder 2), -PO(OR¹³)₂, -NR¹³OR¹⁹, -SO₃H oder -C(O)R¹⁴ bedeuten, worin
R⁷ ein Wasserstoffatom, eine C₁₋₆-Alkyl- oder C₁₋₆-Alkanoylgruppe,
R8 ein Wasserstoffatom, eine C₁₋₆Alkyl-, eine C₁₋₆-Alkanoyl- oder gegebenenfalls substituierte Benzoylgruppe oder einen -CH₂C(O)R¹⁵-Rest (mit R¹⁵ in der Bedeutung von Wasserstoff, einer C₁₋₆-Alkyl-, einer C₁₋₆-Alkoxy- oder einer Hydroxy-Gruppe oder des Restes -NR¹⁶R¹⁷),
R⁹ und R¹⁰ unabhängig voneinander ein Wasserstoffatom, eine C₁₋₄-Alkyl-, C₁₋₄-Alkanoyl- oder eine gegebenenfalls durch Hydroxy oder C₁₋₄-Alkoxy substituierte Benzyl-oder Benzoylgruppe,
R¹¹ eine C₁₋₆-Alkoxygruppe, wobei beide Reste R¹¹ unter Einschluß der CH-Gruppe gegebenenfalls ein cyclisches Acetal bilden,
R¹² eine C₁₋₆-Alkylgruppe, wobei n die Bedeutung von 1 oder 2 zukommt,
R¹³ und R¹⁹ unabhängig voneinander ein Wasserstoffatom oder eine C₁₋₃-Alkylgruppe,
R¹⁴ ein Wasserstoff- oder Halogenatom, eine Hydroxy-, eine C₁₋₆-Alkyl-, eine C₁₋₆-Alkoxy- oder gegebenenfalls durch Hydroxy oder c₁₋₄-Alkoxy substituierte Phenoxy- oder Benzyloxygruppe oder den Rest -NR¹⁶R¹⁷ mit R¹⁶ und R¹⁷ unabhängig voneinander in der Bedeutung Wasserstoffatom, C₁₋₄-Alkylgruppe oder gegebenenfalls durch Hydroxy oder C₁₋₄-Alkoxy substituierte Benzylgruppe darstellen,
mit dem Proviso, daß R⁶ ≠ H, CO₂H, CO₂Alkyl, Alkyl, CN, Tetrazol, wenn R³ und R⁵ = tert.-Butyl und E mit dem linken Phenylkern verknüpft ist, und daß R⁶ ≠ CO₂H, wenn R¹ bis R³ und R⁵ = H und E mit dem rechten Phenylkern verknüpft ist, und daß R¹ bis R⁵ nicht alle gleichzeitig Wasserstoff darstellen,
sowie gegebenenfalls deren physiologisch verträgliche Salze.

2. Diphenylheteroalkylderivate der Formel I gemäß Anspruch 1, dadurch gekennzeichnet, daß R⁶ einen der Reste -CH₂OR⁷, -OR⁸, -SR¹⁰, -SO₂R¹², -SO₃H, -PO(OR¹³)₂ oder -C(O)R¹⁴ bedeutet.

3. Diphenylheteroalkylderivate der Formel I gemäß Anspruch 1, dadurch gekennzeichnet, daß
E für ein Sauerstoff- oder Schwefelatom oder die NH-Gruppe steht,
R³ und R⁵ jeweils für verzweigte C₃₋₆-Alkylgruppen mit R⁴ in der Bedeutung von Wasserstoff oder eines Restes -OR⁷ stehen, und
R⁶ einen der Reste -CH₂OR⁷, -OR⁸, -SR¹⁰, -SO₂R¹², -SO₃H, -PO(OR¹³)₂ oder -C(O)R¹⁴ bedeutet.

4. Verfahren zur Herstellung der Diphenylheteroalkylderivate der Formel I gemäß einem der Ansprüche 1 bis 4, dadurch gekennzeichnet, daß man
a)
- falls A eine -O-CH₂- oder -O-CH(CH₃)-Gruppe bedeutet - Phenole der Formel II 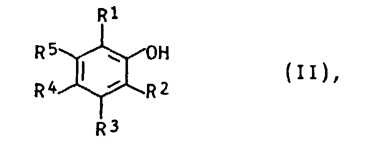 in der R¹-R⁵ die in den Ansprüchen 1 bis 4 angegebenen Bedeutungen haben, mit Benzylderivaten der Formel III 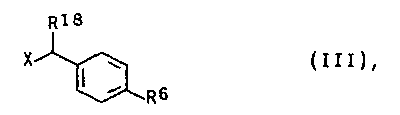 in der R¹⁸ ein Wasserstoffatom oder eine Methylgruppe, X eine nukleofuge Abgangsgruppe darstellt, in Gegenwart einer Base umsetzt, oder
b)
- falls A eine -CH₂-O- oder -CH(CH₃)-O-Gruppe bedeutet - Phenole der Formel IV 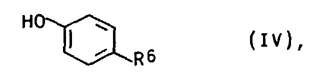 in der R⁶ die in den Ansprüchen 1 bis 4 angegebene Bedeutung hat, mit Benzylderivaten der Formel V 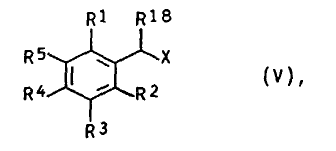 in der R¹ bis R⁵, R¹⁸ und X jeweils die genannten Bedeutungen haben, in Gegenwart einer Base umsetzt, oder
c)
- falls A eine -S(O)ₙ-CH₂- oder -S(O)ₙ-CH(CH₃)-Gruppe bedeutet-Thiophenole der Formel VI 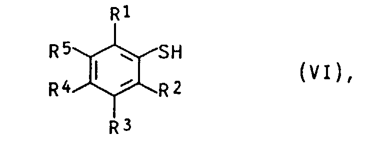 in der R¹ bis R⁵ die in den Ansprüchen 1 bis 4 angegebenen Bedeutungen haben mit Benzylderivaten der Formel III wie unter a) angegeben zu Thioethern (n=0) umsetzt und diese gegebenenfalls mit einem Oxidationsmittel in die korrespondierenden Sulfoxide (n=1) oder Sulfone (n=2) überführt, oder
d)
- falls A eine -CH₂-S(O)ₙ- oder -CH(CH₃)-S(O)ₙ-Gruppe bedeutet - Thiophenole der Formel VII 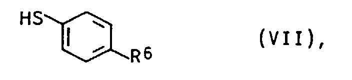 in der R⁶ die in den Ansprüchen 1 bis 4 angegebene Bedeutung hat, mit Benzylderivaten der Formel V wie unter b) angegeben zu Thioethern (n=0) umsetzt und diese gegebenenfalls mit einem Oxidationsmittel in die korrespondierenden Sulfoxide (n=1) oder Sulfone (n=2) überführt, oder
e)
- falls A eine -NR⁷CH₂- oder -NR⁷CH(CH₃)-Gruppe bedeutet - entweder
Aniline der Formel VIII 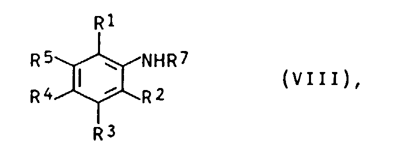 in der R¹ bis R⁵ und R⁷ die in den Ansprüchen 1 bis 4 angegebenen Bedeutungen haben, mit Benzylderivaten der Formel III wie unter a) angegeben umsetzt, oder
Aniline der Formel VIII mit Carbonylverbindungen der Formel IX 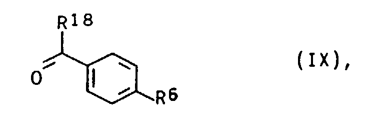 in der R⁶ und R¹⁸ die genannten Bedeutungen haben, in Gegenwart eines Reduktionsmittels umsetzt, oder
f)
- falls A eine -CH₂NR⁷- oder -CH(CH₃)NR⁷-Gruppe bedeutet - entweder
Aniline der Formel X 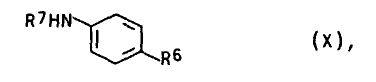 in der R⁶ und R⁷ die in den Ansprüchen 1 bis 4 angegebenen Bedeutungen haben, mit Benzylderivaten der Formel V wie unter b) angegeben umsetzt, oder
Aniline der Formel X mit Carbonylverbindungen der Formel XI 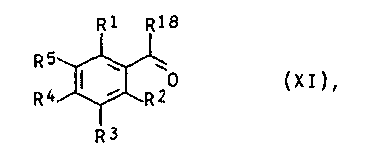 in der R¹-R⁵ und R¹⁸ die genannten Bedeutungen haben, in Gegenwart eines Reduktionsmittels umsetzt und die so erhaltenen Verbindungen gegebenenfalls nach Standardmethoden in weitere Verbindungen der Formel I und gegebenenfalls ihre physiologisch verträglichen Salze umwandelt.

5. Arzneimittel zur topischen Anwendung, das als Wirkstoff 0,001 bis 1 Gew.% eines der Diphenylheteroalkylderivate der Formel I gemäß einem der Ansprüche 1 bis 3 neben üblichen galenischen Hilfsstoffen enthält.

6. Arzneimittel zur systemischen Anwendung, das neben üblichen galenischen Hilfsmitteln als Wirkstoff pro Einzeldosis 0,1 bis 250 mg eines der Diphenylheteroalkylderivate der Formel I gemäß einem der Ansprüche 1 bis 3 neben üblichen galenischen Hilfsstoffen enthält.

7. Kosmetische Zubereitung, die neben üblichen kosmetischen Hilfsstoffen 0,001 bis 1 Gew.% eines der Diphenylheteroalkylderivate der Formel I gemäß einem der Ansprüche 1 bis 3 enthält.

8. Arzneimittel oder Kosmetikum nach Anspruch 5 oder 7 zur Behandlung von Akne oder Psoriasis oder anderen mit pathologisch veränderter Verhornung einhergehenden dermatologischen Erkrankungen sowie von Ekzemen, Warzen, Vitiligo, von durch UV-Licht oder iatrogen bedingten Schädigungen der Haut sowie von Präkanzerosen und Tumoren und trockenen Augen und anderen Corneopathien.

9. Arzneimittel nach Anspruch 6 zur Behandlung von rheumatischen und arthritischen Erkrankungen.

## Claims

1. A diphenylheteroalkyl derivative of the formula I 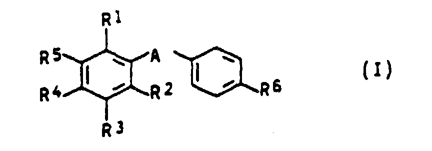 where A is an -E-CH₂- or 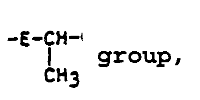 where E can be linked to the phenyl nucleus on the left or on the right and is an oxygen atom, an -S(O)ₙ- or -NR⁷- group (with n meaning 0, 1 or 2),
R¹, R² and R³ are, independently of one another, a hydrogen or halogen atom, a C₁-C₆-alkyl group or an OR⁷ group, one of the three radicals also a nitro group,
R⁴ and R⁵ are, independently of one another, a hydrogen atom, an OR⁷ group or a C₁₋₆-alkyl group, where R⁴ is a branched C₃₋₆-alkyl or -alkoxy group when R¹ to R³ are hydrogen, and R⁴ (alone) or R³ and R⁵ (both) are each a branched C₃₋₆-alkyl group when R⁶ is a hydrogen atom, a methyl, nitro, OH or SH group,
R⁶ is a hydrogen atom, a methyl, a nitro or nitrile group, the tetrazolyl radical or the radicals -CH₂OR⁷, -OR⁸, -NR⁹R¹⁰, -CH₂NR⁹R¹⁰, -SR¹⁰, CH(R¹¹)₂, -S(O)ₙR¹² (with n=1 or 2), -PO(OR¹³)₂, -NR¹³OR¹⁹, -SO₃H or -C(O)R¹⁴, where R⁷ is a hydrogen atom, a C₁₋₆-alkyl or C₁₋₆-alkanoyl group, R⁸ is a hydrogen atom, a C₁₋₆-alkyl, a C₁₋₆-alkanoyl or unsubstituted or substituted benzoyl group or a -CH₂C(O)R¹⁵ radical (with R¹⁵ meaning hydrogen, a C₁₋₆-alkyl, a C₁₋₆-alkoxy or a hydroxyl group or the radical -NR¹⁶R¹⁷),
R⁹ and R¹⁰ are, independently of one another, a hydrogen atom, a C₁₋₄-alkyl, C₁₋₄-alkanoyl group or a benzyl or benzoyl group which is unsubstituted or substituted by hydroxyl or C₁₋₄-alkoxy,
R¹¹ is a C₁₋₆-alkoxy group, it being possible for the two R¹¹ radicals to form, with inclusion of the CH group, a cyclic acetal,
R¹² is a C₁₋₆-alkyl group, where n has the meaning of 1 or 2,
R¹³ and R¹⁹ are, independently of one another, a hydrogen atom or a C₁₋₃-alkyl group,
R¹⁴ is a hydrogen or halogen atom, a hydroxyl, a C₁₋₆-alkyl, a C₁₋₆-alkoxy group or phenoxy or benzyloxy group which is unsubstituted or substituted by hydroxyl or C₁₋₄-alkoxy, or the radical -NR¹⁶R¹⁷ with R¹⁶ and R¹⁷ meaning, independently of one another, hydrogen atom, C₁₋₄-alkyl group or benzyl group which is unsubstituted or substituted by hydroxyl or C₁₋₄-alkoxy,
with the proviso that R⁶ ≠ H, CO₂H, CO₂alkyl, alkyl, CN, tetrazole when R³ and R⁵ = tert-butyl and E is linked to the phenyl nucleus on the left, and that R⁶ ≠ CO₂H when R¹ to R³ and R⁵ = H and E is linked to the phenyl nucleus on the right, and that R¹ to R⁵ are not all hydrogen at the same time,
and the physiologically tolerated salts thereof where appropriate.

2. A diphenylheteroalkyl derivative of the formula I as claimed in claim 1, wherein R⁶ is one of the radicals -CH₂OR⁷, -OR⁸, -SR¹⁰, -SO₂R¹², -SO₃H, -PO(OR¹³)₂ or -C(O)R¹⁴.

3. A diphenylheteroalkyl derivative of the formula I as claimed in claim 1, wherein
E is an oxygen or sulfur atom or the NH group,
R³ and R⁵ are each branched C₃₋₆-alkyl groups with R⁴ meaning hydrogen or a radical -OR⁷, and
R⁶ is one of the radicals -CH₂OR⁷, -OR⁸, -SR¹⁰,-SO₂R¹², -SO₃H, -PO(OR¹³)₂ or -C(O)R¹⁴.

4. A process for preparing the diphenylheteroalkyl derivatives of the formula I as claimed in any of claims 1 to 4, which comprises
a) if A is an -O-CH₂- or -O-CH(CH₃)- group, reacting phenols of the formula II 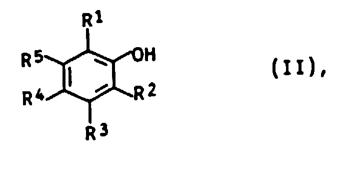 where R¹-R⁵ have the meanings stated in claims 1 to 4, with benzyl derivatives of the formula III 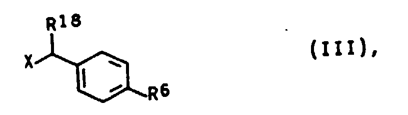 where R¹⁸ is a hydrogen atom or a methyl group, X is a nucleofugic leaving group, in the presence of a base, or
b) if A is a -CH₂-O- or -CH(CH₃)-O- group, reacting phenols of the formula IV 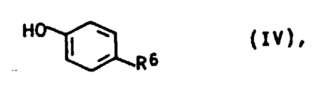 where R⁶ has the meanings stated in claims 1 to 4, with benzyl derivatives of the formula V 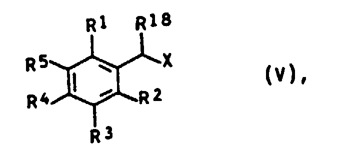 where R¹ to R⁵, R¹⁸ and X each have the said meanings, in the presence of a base, or
c) if A is an -S(O)ₙ-CH₂- or -S(O)ₙ-CH(CH₃)- group, reacting thiophenols of the formula VI 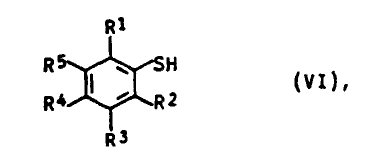 where R¹ to R⁵ have the meanings stated in claims 1 to 4, with benzyl derivatives of the formula III as indicated under a) to give thioethers (n=0), and converting the latter where appropriate with an oxidizing agent into the corresponding sulfoxides (n=1) or sulfones (n=2), or
d) if A is an -CH₂-S(O)ₙ- or -CH(CH₃)-S(O)ₙ- group, reacting thiophenols of the formula VII 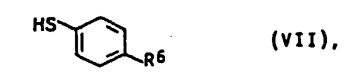 where R⁶ has the meanings stated in claims 1 to 4, with benzyl derivatives of the formula V as indicated under b) to give thioethers (n=0), and converting the latter where appropriate with an oxidizing agent into the corresponding sulfoxides (n=1) or sulfones (n=2), or
e) if A is an -NR⁷CH₂- or -NR⁷CH(CH₃)- group, either reacting anilines of the formula VIII 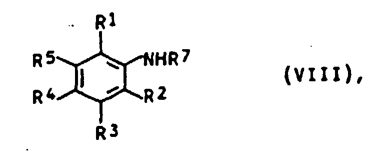 where R¹ to R⁵ and R⁷ have the meanings stated in claims 1 to 4, with benzyl derivatives of the formula III as indicated under a), or
reacting anilines of the formula VIII with carbonyl compounds of the formula IX 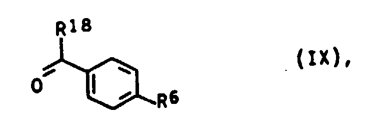 where R⁶ and R¹⁸ have the stated meanings, in the presence of a reducing agent, or
f) if A is a -CH₂NR⁷- or -CH(CH₃)NR⁷- group, either reacting anilines of the formula X 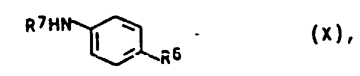 where R⁶ and R⁷ have the meanings stated in claims 1 to 4, with benzyl derivatives of the formula V as indicated under b), or
reacting anilines of the formula X with carbonyl compounds of the formula XI 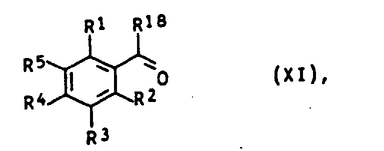 where R¹-R⁵ and R¹⁸ have the stated meanings, in the presence of a reducing agent, converting the compounds obtained in this way where appropriate by standard methods into other compounds of the formula I and, where appropriate, their physiologically tolerated salts.

5. A drug for topical use which comprises as active substance from 0.001 to 1 % by weight of one of the diphenylheteroalkyl derivatives of the formula I as claimed in any of claims 1 to 3 in addition to conventional pharmaceutical ancillary substances.

6. A drug for systemic use which, in addition to conventional pharmaceutical aids, comprises as active substance per single dose from 0.1 to 250 mg of one of the diphenylheteroalkyl derivatives of the formula I as claimed in any of claims 1 to 3 in addition to conventional pharmaceutical ancillary substances.

7. A cosmetic preparation which, in addition to conventional cosmetic ancillary substances, comprises from 0.001 to 1 % by weight of one of the diphenylheteroalkyl derivatives of the formula I as claimed in any of claims 1 to 3.

8. A drug or cosmetic as claimed in claim 5 or 7 for the treatment of acne or psoriasis or other dermatological disorders associated with a pathological change in keratinization, and of eczemas, warts, vitiligo, skin damage caused by UV light or iatrogenically, and of precanceroses and tumours and dry eyes and other corneopathies.

9. A drug as claimed in claim 6 for the treatment of rheumatic and arthritic disorders.

## Revendications

1. Dérivés de diphénylhétéroalkyle de formule I 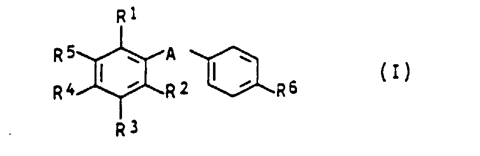 où les symboles représentent les atomes ou groupes suivants:
A un groupe -E-CH₂- ou 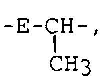 tandis que E peut être relié au noyau phényle gauche ou droit et désigne un atome d'oxygène, un groupe -S(O)ₙ- ou un groupe -NR⁷- (avec n valant 0, 1 ou 2),
R¹, R² et R³, indépendamment l'un de l'autre, un atome d'hydrogène ou d'halogène, un groupe alkyle en C₁-C₆ ou un groupe -OR⁷, et l'un des trois restes également un groupe nitro,
R⁴ et R⁵, indépendamment l'un de l'autre, un atome d'hydrogène, un groupe -OR⁷ ou un groupe alkyle en C₁₋₆, tandis que R⁴ représente un groupe alkyle ou alcoxy en C₃₋₆ ramifié, lorsque R¹ à R³ sont l'hydrogène, et R⁴ (seulement) ou R³ et R⁵ (tous les deux) représentent à chaque fois un groupe alkyle en C₃₋₆ ramifié, lorsque R⁶ est un atome d'hydrogène, un groupe méthyle, nitro, OH ou SH,
R⁶ un atome d'hydrogène, un groupe méthyle, un groupe nitro ou un groupe nitrile, le reste tétrazolyle ou les restes -CH₂OR⁷, -OR⁸, -NR⁹R¹⁰, -CH₂NR⁹R¹⁰, -SR¹⁰, CH(R¹¹)₂, -S(O)ₙR¹² (avec n = 1 ou 2), -PO(OR¹³)₂, -NR¹³OR¹⁹, -SO₃H ou -C(O)R¹⁴, tandis que les symboles représentent les atomes ou groupes suivants:
R⁷ un atome d'hydrogène, un groupe alkyle en C₁₋₆ ou un groupe alcanoyle en C₁₋₆,
R⁸ un atome d'hydrogène, un groupe alkyle en C₁₋₆, un groupe alcanoyle en C₁₋₆ ou un groupe benzoyle, éventuellement substitué, ou un reste -CH₂C(O)R¹⁵ (avec R¹⁵ désignant l'hydrogène, un groupe alkyle en C₁₋₆, un groupe alcoxy en C₁₋₆ ou un groupe hydroxy ou le reste -NR¹⁶R¹⁷),
R⁹ et R¹⁰, indépendamment l'un de l'autre, un atome d'hydrogène, un groupe alkyle en C₁₋₄, un groupe alcanoyle en C₁₋₄ ou un groupe benzyle ou benzoyle éventuellement substitué par un reste hydroxy ou alcoxy en C₁₋₄,
R¹¹ un groupe alcoxy en C₁₋₆, tandis que deux restes R¹¹ forment éventuellement un cycle acétal en y incluant le groupe CH,
R¹² un groupe alkyle en C₁₋₆, tandis que n vaut alors 1 ou 2,
R¹³ et R¹⁹, indépendamment l'un de l'autre, un atome d'hydrogène ou un groupe alkyle en C₁₋₃,
R¹⁴ un atome d'hydrogène ou d'halogène, un groupe hydroxy, un groupe alkyle en C₁₋₆, un groupe alcoxy en C₁₋₆ ou un groupe phénoxy ou benzyloxy éventuellement substitué par un reste hydroxy ou alcoxy en C₁₋₄, ou le reste -NR¹⁶R¹⁷, avec R¹⁶ et R¹⁷ représentant indépendamment l'un de l'autre un atome d'hydrogène, un groupe alkyle en C₁₋₄ ou un groupe benzyle éventuellement substitué par un reste hydroxy ou alcoxy en C₁₋₄,
avec pour condition que R⁶ ≠ H, CO₂H, CO₂alkyle, alkyle, CN, tétrazole, lorsque R³ et R⁵ = tert-butyle et que E est relié au noyau phényle gauche, et pour condition que R⁶ ≠ CO₂H, lorsque R¹ à R³ et R⁵ = H et que E est relié au noyau phényle droit, et avec pour condition que R¹ à R⁵ ne représentent pas tous simultanément l'hydrogène,
ainsi qu'éventuellement leurs sels physiologiquement acceptables.

2. Dérivés de diphénylhétéroalkyle de formule I selon la revendication 1, caractérisés par le fait que R⁶ représente l'un des restes -CH₂OR⁷, -OR⁸, -SR¹⁰, -SO₂R¹², -SO₃H, -PO(OR¹³)₂ ou -C(O)R¹⁴.

3. Dérivés de diphénylhétéroalkyle de formule I selon la revendication 1, caractérisés par le fait que
E désigne un atome d'oxygène ou de soufre ou le groupe NH,
R³ et R⁵ désignent chacun des groupes alkyle en C₃₋₆ ramifiés, tandis que R⁴ représente l'hydrogène ou un reste -OR⁷, et
R⁶ représente l'un des restes -CH₂OR⁷, -OR⁸, -SR¹⁰, -SO₂R¹², -SO₃H, -PO(OR¹³)₂ ou -C(O)R¹⁴.

4. Procédé pour la préparation de dérivés de diphénylhétéroalkyle de formule I selon l'une quelconque des revendications 1 à 4, caractérisé par le fait que
a)
- dans le cas où A représente un groupe -O-CH₂- ou -O- CH(CH₃)-, on fait réagir en présence d'une base des phénols de formule II 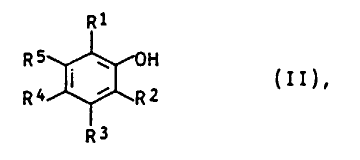 où R¹-R⁵ ont les significations indiquées dans les revendications 1 à 4, avec des dérivés de benzyle de formule III 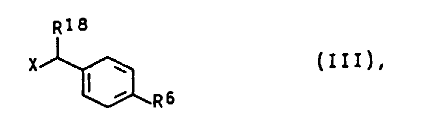 où R¹⁸ représente un atome d'hydrogène ou un groupe méthyle, X représente un groupe-éliminable nucléofuge, ou
b)
- dans le cas où A représente un groupe -CH₂-O- ou -CH(CH₃)-O-, on fait réagir en présence d'une base des phénols de formule IV 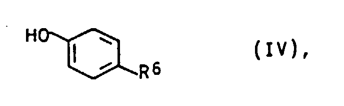 où R⁶ a la signification indiquée dans les revendications 1 à 4, avec des dérivés de benzyle de formule V 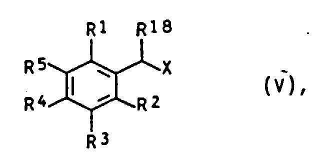 où R¹ à R⁵, R¹⁸ et X ont chacun les significations susdites, ou
c)
- dans le cas où A représente un groupe -S(O)ₙ-CH₂- ou -S(O)ₙ-CH(CH₃), on fait réagir des thiophénols de formule VI 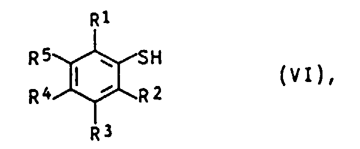 où R¹ à R⁵ ont les significations indiquées dans les revendications 1 à 4, avec des dérivés de benzyle de formule III tels qu'indiqué sous a) pour former des thioéthers (n = 0) et on convertit ceux-ci éventuellement avec un agent d'oxydation en les sulfoxydes (n = 1) ou les sulfones (n = 2) correspondants, ou
d)
- dans le cas où A représente un groupe -CH₂-S(O)ₙ- ou -CH(CH₃)-S(O)ₙ-, on fait réagir des thiophénols de formule VII 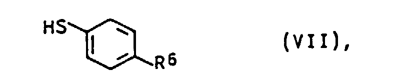 où R⁶ a la signification indiquée dans les revendications 1 à 4, avec des dérivés de benzyle de formule V comme indiqué sous b) pour former des thioéthers (n = 0) et on convertit ceux-ci éventuellement avec un agent d'oxydation en les sulfoxydes (n = 1) ou les sulfones (n = 2) correspondants, ou
e)
- dans le cas où A représente un groupe -NR⁷CH₂- ou -NR⁷CH(CH₃),
soit on fait réagir de l'aniline de formule VIII 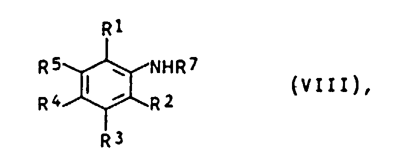 où R¹ à R⁵ et R⁷ ont les significations indiquées dans les revendications 1 à 4, avec des dérivés de benzyle de formule III comme indiqué sous a),
soit on fait réagir de l'analine de formule VIII avec des composés carbonylés de formule IX 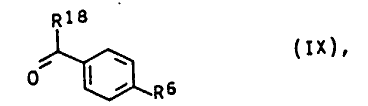 où R⁶ et R¹⁸ ont les significations indiquées, en présence d'un agent de réduction, ou
f)
- dans le cas où A représente un groupe -CH₂NR⁷- ou -CH(CH₃)NR⁷-,
soit on fait réagir de l'aniline de formule X 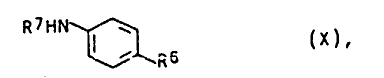 où R⁶ et R⁷ ont les significations indiquées dans les revendications 1 à 4, avec des dérivés de benzyle de formule V comme indiqué sous b),
soit on fait réagir de l'aniline de formule X avec des composés carbonylés de formule XI 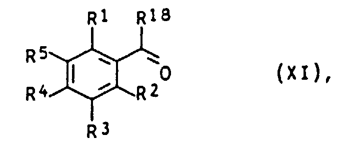 où R¹-R⁵ et R¹⁸ ont les significations indiquées, en présence d'un agent de réduction, et on transforme les composés ainsi obtenus éventuellement selon des méthodes standard en d'autres composés de formule I et éventuellement en leurs sels physiologiquement acceptables.

5. Médicament pour application topique, qui contient, en tant qu'agent actif, 0,001 à 1% en poids d'un des dérivés de diphénylhétéroalkyle de formule I selon l'une quelconque des revendications 1 à 3, ainsi que des adjuvants galéniques classiques.

6. Médicament pour application systémique, qui contient, en tant qu'agent actif, par dose unitaire 0,1 à 250 mg d'un des dérivés de diphénylhétéroalkyle de formule I selon l'une quelconque des revendications 1 à 3, ainsi que des adjuvants galéniques classiques.

7. Préparation cosmétique, qui contient, outre des adjuvants cosmétiques classiques, 0,001 à 1% en poids d'un des dérivés de diphénylhétéroalkyle de formule I selon l'une quelconque des revendications 1 à 3.

8. Médicament ou cosmétique selon l'une des revendications 5 ou 7 pour le traitement de l'acné ou du psoriasis ou d'autres affections dermatologiques provoquant un racornissement pathologiquement modifié, ainsi que d'eczémas, de verrues, de vitiligos, d'altérations de la peau provoquées par la lumière UV ou iatrogènes, ainsi que d'atteintes précancéreuses et de tumeurs et de syndromes de l'oeil sec et d'autres cornéopathies.

9. Médicament selon la revendication 6 pour le traitement d'affections rhumatismales et arthritiques.
